(19) Europäisches Patentamt
European Patent Office
Office européen des brevets

(11) **EP 1 613 397 B1**

(12) **EUROPEAN PATENT SPECIFICATION**

(45) Date of publication and mention
of the grant of the patent:
**04.05.2011 Bulletin 2011/18**

(21) Application number: **04723273.1**

(22) Date of filing: **25.03.2004**

(51) Int Cl.:
*A61P 31/04* *(2006.01)*    *A61K 35/74* *(2006.01)*

(86) International application number:
**PCT/IB2004/000885**

(87) International publication number:
**WO 2004/087189 (14.10.2004 Gazette 2004/42)**

(54) **USE OF BACTERIOCIN FOR THE AMELIORATION OF DIGESTIVE FUNCTIONALITY**

VERWENDUNG VON BACTERIOCIN ZUR VERBESSERUNG DER VERDAUUNGSFUNKTION

UTILISATION DE LA BACTERIOCINE POUR AMELIORER LA FONCTIONNALITE DIGESTIVE

(84) Designated Contracting States:
**AT BE BG CH CY CZ DE DK EE ES FI FR GB GR HU IE IT LI LU MC NL PL PT RO SE SI SK TR**

(30) Priority: **01.04.2003 PCT/IT03/00193**

(43) Date of publication of application:
**11.01.2006 Bulletin 2006/02**

(73) Proprietor: **AVIP S.R.L.**
**40124 Bologna (BO) (IT)**

(72) Inventors:
• **Piva, Andrea**
**40123 Bologna (IT)**
• **Casadei, Gabriele**
**47020 Cesena (IT)**

(74) Representative: **HOFFMANN EITLE**
**Patent- und Rechtsanwälte**
**Arabellastraße 4**
**81925 München (DE)**

(56) References cited:
**EP-A- 0 384 319    EP-A- 0 466 244**
**WO-A-97/29645**

• PIVA A ET AL: "PEDIOCIN A, A BACTERIOCIN PRODUCED BY PEDIOCOCCUS PENTOSACEUS FBB61" MICROBIOLOGY, SOCIETY FOR GENERAL MICROBIOLOGY, READING, GB, vol. 140, no. PART 4, April 1994 (1994-04), pages 697-702, XP001180469 ISSN: 1350-0872
• LEWUS C B ET AL: "FURTHER CHARACTERIZATION OF BACTERIOCINS PLANTARICIN BN, BAVARICIN MN AND PEDIOCIN A" PROCEEDINGS INSTITUTE OF ENVIRONMENTAL SCIENCES, XX, XX, vol. 6, no. 2, 1992, pages 153-174, XP008012433

**Description**

TECHNICAL FIELD

The subject of the present invention is the use of bacteriocins and/or their produce strains for the amelioration of digestive functionality and for the amelioration of gastrointestinal tract conditions in monogastric organism species.

STATE OF THE ART

Bacteria belonging to the genus *Pediococcus* are known. Bacteria belonging to the genus *Pediococcus* are homofermenting, facultative anaerobes non encapsulated. Bacteria belonging to the genus *Pediococcus* are chemoorganotroph, therefore they have complex nutritive requirements, they don't have proteolytic activity and are saprophytae of many fresh and fermenting plants. Their economical importance in the transformation and conservation of plant products, meat and their contribution in the development of cheese flavours make them a particularly attractive genus from the industrial point of view. Bacteria belonging to the genus *Pediococcus* have been isolated from bovine rumen and chick cecum. Eight species, depending on their to sensitivity temperature, pH and NaCl may be identified.

P. cerevisiae species has been eliminated and previously thus indicated strains have met partly in P. damnosus species and partly in P. pentosaceus species. Bacteria belonging to this latter class have their natural habitat in plants and exhibit development temperatures lower than 50°C (for FBB61 P. pentosaceus, the optimum is established at 35°C), in contrast to thermophile pediococci which grow well at 50°C, they have smaller cell sizes ($0,5-0,8$ $\mu$m) and are groupped in P. acidilactici species.

It is known that one of the lactic bacteria features, which make them particularly interesting for a possible probiotic use, is their ability in competing with other bacteria for the same ecological niche, and to prevail. This ability is due to organic acids and metabolism by-products high concentrations production, assisted by substances with antimicrobial action proteic in nature, designated as "bacteriocins".

It is known that FBB61 *Pediococcus* pentosaceus microorganism is responsible for the production of a proteinaceous antimicrobial substance (bacteriocin) designated as pediocin A.

It is known that the lactic bacteria ability in competing with other bacteria for the same ecological niche and to prevail, besides of being due to organic acid production and low pH level tolerance, it is sometimes strengthened by the production of antimicrobial substances proteinaceous in nature. It is widely recognized that these proteins or peptides with antimicrobial action, designated with the term "bacteriocins" help in a decisive way to survival or dominance of certain strains in microbial ecosystems, such as foods or the digestive tract.

Examples of bacteriocin productions exist in all known bacterial species and generally they only have an antagonistic activity against those species covering the same ecological niche or those philogenetically close to the producer microorganism. Lantionine-containing peptides such as nisin and subtilin constitute an exception to this rule, as they generally show antimicrobial activity against Gram +.

The very long positive use of these bacteria probably resides in the production of these bacteriocins till now studied for the purpose of extending foods preservability and improving hygienic features with reference to pathogenic microorganism development.

International Patent Application WO-A-9729645 discloses a combination of Pediococcus pentosaceus with at least two bacteriocins for the treatment of bacterial infections in the stomach and the large and small intestinal tract. However, WO-A-9729645 is silent on compositions encompassing Pediocin A or an analogue thereof and its bacterial producer strains selected from at least one of: Pediococcus pentosaceus FBB61, i.e. ATCC 43200; Pediococcus pentosaceus FBB63; Pediococcus pentosaceus L7230, i.e. ATCC43201.

Therefor, it is known that bacteriocins have been so far studied, produced and purified for their possible use like end products and/or raw materials preservatives by virtue of their marked antimicrobial abilities towards impairing and/or harmful microorganisms. However, use of Bacteriocins as intestinal environment modulators and sanitary conditions enhancers of the intestine has not been investigated so far.

DESCRIPTION OF THE INVENTION

This invention relates to a new use of bacteriocins and their producer strains as gastrointestinal tract conditions enhancers of the monogastric organism species here enclosed: humans, swines, rabbits, horses, poultry also wild, sheep, goats, felids, canids and non functional ruminants. The addition of a bacteriocin and its producer bacterium, a lactic bacterium, in this case pediocin A and FBB61 *Pediococcus pentosaceus*, FBB63 *Pediccoccus pentosaceus* and its bacteriocins, L 7230 *Pediococcus pentosaceus* and its bacteriocins, to diet produces positive effects on the organism thanks to a better general arrangement of the gastrointestinal tract.

Lactic bacteria, including lactobacilli and bifid back teria, regarded as GRAS (Generally Recognised as Safe), which are introduced in the diet and/or administrated to organism lead to the production of lactic acid inside the gastrointestinal tract, which determines an intestinal pH reduction. This low pH is inhospitable for several undesired microorganisms (including human and animal phatogens) that cannot survive in so acidic conditions. Consequently, the invention develops a better microbic intestinal balance. Colonization of these bacteria is promoted by the production of antimicrobial proteinaceous molecules, called bacteriocins, which are able to act and therefore to be used in any way, alone as well, having a self-activity, without the compulsory presence of producer strain. A further advantage of the invention result from the reduction of oncogenic compounds due to the proliferation of beneficial bacteria, either producer strains of said bacteriocins or other beneficial bacterial strains not affected by the antimicrobial action of antimicrobial proteins. As it has been shown by in vitro fermentation systems, such effects are pointed out in the reduction of cresol production, a tiroxina metabolite which is considered responsible for the onset of skin and epatic tumors in mouse, as well as a depressing factor of the animal growth, e.g. in swines. Moreover, stilt into in vitro systems, the presence of bacteriocins and their producer bacterial strains leads to a considerable increase of polyamine productions of bacterial origin, especially putrescine and spermidine, usually known as very important energetic sources for gastroenteric cells. The presence of high quantities of these molecules represents not only an important condition especially for young animal, where the proliferation and renewal rate of intestinal cells is higher than every other cell of the organism. The above discussed remarks, pointed out in vitro, show an actual modulation action of intestinal metabolic processes by the bacterial component, whose consequences find then expression in a higher hygiene and health of gatroenteric tract, therefore a better animal's state of health.

The introduction of bacteriocins and their producer strains into diet leads so a better morphofunctional arrangement of intestinal wall with an increase of ephitelial surface deputed to the absorption of nutrients without affecting its integrity and consequently the functionality. These remarks together with a renewed and improved intestinal microbial balance and consequent metabolic modifications affect an optimal organism state of health and a better efficiency of nutrients use. The efficiency of the invention also results from the interaction of cited bacteriocins with intestinal mucosa structures. More precisely, although not being related to any specific mechanism, the descripted bacteriocins, when introduced with diet or produced in situ by respective producers bacterial strains play their action both in intestinal lumen and by being incorporate in mucous layer which cover the intestinal structures, and remaining active therein they play a protection role against pathogenic bacteria attack, both human and animal, preventing the mucosa attack and the harmful action with consequent reduction of morbidity and onset of gastrointestinal disorders, detrimental to organism. Therefore, the incorporation in the intestinal mucus preserve the functional integrity and preserve the bacteriocins from digestive processes attacks. A surprising aspect of this invention is the gastroenteric environment modulation from all microbiologic, metabolic, hygienic, sanitary and prophylactic aspects, with an amelioration of digestive tract health conditions. Therefore, the invention is particularly useful when bacteriocins and their producer strains are employed as additive, integrator, supplement into diet for all the above mentioned species.

The use of invention further includes its employement in any extent in the prevention and prophylaxis of the pathologies by clostridia in any of the above mentioned species, as well as a support to any intervention before, during and/or after the onset of such pathologies.

In a preferred embodiment, this invention refers to a new use of bacteriocins and their producer strains as enhancers of gastrointestinal tract conditions of monogastric organism species. Bacteriocins and more suitable bacterial strains are FBB61 *Pediococcus pentosaceus,* FBB63 *Pediococcus pentosaceus*, L 7230 Pedio*coccus pentosaceus* (ATCC 43201) as well as all bacteriocins produced thereof and/or analogous molecules to pediocin A.

When ingested, microorganisms and their bacteriocins lead to an amelioration of food efficiency with a better use of nutrients and an improved microbiological, metabolic, sanitary condition by way of: 1) lactic acid production with pH decrease and onset of adverse conditions for harmful and/or pathogenic bacteria, 2) gastroenteric tract colonization by beneficial bacteria at harmful microorganis' cost, 3) marked bacteriocins antimicrobial action against gram + bacteria, including Clostridia, adverse to organism, 4) metabolism modulation with reduction of the production of oncogenic products (cresol), 5) metabolism modulation with increase of the polyamines presence (putrescine and spermidine) into intestinal lumen, that function as important energetic sources for gastroenteric structures, 6) increase of.intestinal absorptive surface and maintenance of its proper functionality, 7) adhesion of bacteriocins in the mucus covering gastroenteric mucosa with constitution of a protection barrier against any harmful microorganisms action.

In a preferred embodiment, it is expected the introduction of multiple bacteriocins producer bacteria and the bacteriocins themselves in any useful combination.

The addition and mixing of above mentioned components may occur manually or by using any number and kind of devices fitted for the manufacturing of small and large quantities of liquid or solid feed. If the feed is not particulated in nature, bacteria and bacteriocins may be added at the same time of granulation and/or grinding and/of pulverization of the feed itself. Any method suitable for the feed preparation in any form thereof may be used in order to associate and make possible the introduction of bacteria and bacteriocins in the feed, also by using binding agent, adsorbents, excipients and any other method and/or substance suitable for the purpose.

Similarly, producer bacterial strains of bacteriocins and their bacteriocins may be added to diet or to single raw materials alone or in any combination, any physical form both freeze-dried, liquid, dried, pelletized, chemical and free or protected form with any proper method.

Conveniently, bacteriocins are present in the feed in a quantity from 1 to 10,000 active units (AU) per ml and/or g of feed.

The following examples are only to be intended as an help for a better understanding of the invention.

EXAMPLES

Bacterial strains and growth conditions

During the research they have been employed: FBB61 *Pediococcus pentosaceus* strain (ATCC 43200), as a Pediocin A producer and FBB61-2 Pediococcus pentosaceus strain, isogenetic mutant which does not produce bacteriocin and does not show immunity characters (Imm-), (Daeschel, and Klaenhammer, 1985) as an indicator strain. Those two strains were coltured overnight, in M17 broth medium (Oxoid Ltd, Dasingstoke, UK) + 1% (w/v) glucose at 39°C (Sambrook et al., 1989). Agarized mediums and those used for overlay tests were prepared with the addition of 1,5% (w/v) agar to the liquid medium.

Bacteriocin production

The two FBB61 and FBB61-2 bacterial strains were incubated in a M17 broth + 1% (w/v) glucose at 39°C for 24 hours. The coltures were then centrifuged at 10,000 rpm for 15 minutes and the supernatant collected and filtered (Nalgene Filterware, Nalge Nunc International, NY, USA) through membranes with ø = 0,45 $\mu$m pores. Next, the filtered supernatant was concentrated through dialysis against polyethylene glycol of 20,000 Da molecular weight (PEG 20000, Sigma Chemical, St. Louis, MO, USA). For this operation, dialysis tubes with 12000/14000 MW cut-off (Spectra/Pro 4, Spectrum, Houston, Texas, USA) were employed. The process was carried out at a temperature of 4°C for a whole night. The two' solutions, in which the dialysis tube were dipped, were constantly stirred by a stirrer, in order to promote a better osmotic flow.

Pediocin A purification

The dialyzate of the two coltures FBB61 (Dia+ = dialyzate of producer strain) and FBB61-2 (Dia- = dialyzate of non producer and sensitive strain) was subjected to further dialysis against a 150 mM NaCl, 20 mM. Tris-HCl solution, still at a temperature of 4°C overnight. The pH of the obtained solution was then adjusted at 8,1 by addition of HCl 33%. The obtained solution was further sterilized by way of filtration through 0,45 $\mu$m porosity filters.

The pediocin A purification was carried out by low pressure ion-exchange chromatography.

Five 5 ml each ion-exchange chromatographic columns were mass-mounted and connected through silicone tubes to a peristaltic pump (Minipuls 3, Gilson Italia Srl, Milan, Italy). The thus obtained column was prepared by first passing through it by a start-buffer (20 mM Tris-HCl, pH 8,2), then by a regeneration-buffer (20 mM Tris-HCl + 1 M NaCl, pH 8,2) and then, by a start-buffer2 (20 mM Tris-HCl + 150 mM NaCl, pH 8,2). The volume used for each solution, 5-times than the total of the column, was pumped at a rate of 5 ml/min.

Each sample prepared was charged into the chromatographic column at a rate of 5 ml/min. until column saturation and the obtained residue was collected and frozen. The following fractions separation was obtained by passing through the column 3 volumes of increasing ionic strength NaCl solutions (250 mM, 500 mM, 750 mM, 1 M) buffered at pH 8,1.

Ion-exchange chromatography process was continuously performed inside a proper cooler in order to maintain a temperature of about 4°C.

*In vitro* intestinal fermentations

As it is a matter of processes that occur on the intestinal content of large intestine after digestion and ileal adsorption, it is necessary to simulate such effect on the feed to be employed in the fermentation. The feed, comprised of: corn, 38%; barley, 30%; wheat bran, 15%; soya flour, 12%; herring flour, 2%; vitamins and minerals, 3%, is prepared by enzimatic pre-digestion divided in two moments, according to the first part of Vervaeke et al. methodology (1989).

1st - Gastric digestion step: 2 g of feed (ø of the particles < 1 mm) in 40 ml of 0,2% pepsin 0,075 N HCl solution (E.C. 3.4.23.1, P7000; Sigma Chemical, St. Louis, MO, USA) are incubated at 39°C in the stirred bain-marie for 4 hr.

When first passage solution has been adjusted at pH 7,5 with 1 N NaOH, it begins the

2nd - intestinal digestion step: wherein an equal quantity of buffer, 40 ml each 2 g of feed is added to 1% pancreatin (P1500 Sigma Chemical, St. Louis, MO, USA) and incubated as in the 1st passage.

The buffer was obtained by mixing:

- 500 ml of a solution: 46,5 g $Na_2HPO_4$ + 49,0 g $NaHCO_3$ + 2,35 g of NaCl + 2,85 g of KCl, first dissolved and then adjusted to 1 liter with ion-exchanged water,
- 5 ml of a 6% $MgCl_2$ solution,
- 5 ml of a 4% $CaCl_2$ solution.

Therefore, total 510 ml were then adjusted, still with ion-exchanged water to 2,5 liters and then adjusted at pH 7,5 with 3N HCl (Martillotti et al., 1987).

The pre-digested food was obtained by centrifugation at 3000 x g and 4°C, washed 3 times with ion-exchanged water, intestinal adsorption simulation always eliminated by centrifugation.

The solid residue accumulated on tubes bottom after centrifugation was placed in a stove at 55°C, the required temperature in order to prevent food active principles denaturation, until drying (about 24 hours), then re-grounded and stored sealed in a freezer.

Food analysis before and after pre-digestion

|  | Whole feed | Pre-digested feed |
| --- | --- | --- |
| % humidity | 10,77 | 0,00 |
| % crude protein | 17,59 | 4,19 |
| % crude lipids | 4,10 | 1,98 |
| % crude ashes | 3,73 | 2,95 |
| % NDF | 14,08 | 27,23 |
| % ADF | 4,97 | 11,27 |
| % starch | 49,42 | 50,04 |
| Extraction efficiency 35-40% | | |

Caecal inoculum preparation obtained by slaughtering

1st - Withdrawal step: just after the intestine extraction from the carcass, the cecum was bonded at the level of the ileum-caecal valve and cut. From a little hole at cecum head, the content was flown gently in a nylon vessel, from which air was completely discharged, wand dipped in water at 39°C into a thermal bag, until the preliminary step carried out in a laboratory.

2nd - Laboratory step: the caecal content was measured by pouring it in a graduate, which was held at 39°C before use like all other vessels to be used in direct contact with the inoculum. When the volume was known, it was mixed with the buffer solution in a 1:2 ratio. The buffer composed of: $NaHCO_3$, 49 g; KCl, 2,85 g; $CaCl_2$ x $6H_2O$, 0,395 g; $Na_2HPO_4$ x $12H_2O$, 46,5 g; NaCl, 3,5 g; $MgSO_4$ x 7H20, 0,6 g dissolved in 5 l of ion-exchanged water (McDougall, 1948) was adjusted at pH 6,7 with the addition of 3N HCl. This solution was maintained at 39°C in a bain-marie and sparged with anhydride ($CO_2$) for 20 min before use. The compound was then filtered through 8-layer gauzes in a flask, closed with cotton and gauze and then put again in a bain-marie under $CO_2$ for 10 min. In the vessels with the pre-digested feed, heated at 39°C, the freshly prepared caecal content was poured by way of a cannula connected to a cylinder, after sparging of CO2 on the bottom.

Immediately after, the jars were tight-sealed and dipped in a bain-marie at 39°C, where they remained continuously stirred throughout the fermentation lenght (48 hours).

Fermentation operational diagram:

- Thesis 1, Control: 20 mg flour + 5 ml inoculum
- Thesis 2, BAC +: 20 mg pre-digested feed + 5 ml inoculum + pediocin A equal to a final concentration = 160 AU/ml,
- Thesis 3, BAC-: 20 mg pre-digested feed -+ 5 ml inoculum + same fractions of supernatant elution of FBB61-2 Pediococcus pentosaceus corresponding to FBB61 Pediococcus pentosaceus fractions containing pediocin A. The added quantity produced a proteinaceous concentration equivalent to that produced with pediocin A in thesis 2.

Chemical analysis of feeds and caecal liquid

Diet centesimal analysis was performed according to AOAC procedure (1990), and the fibrous fractions were neutral-washed (NDF), acidic-washed (ADF) and the lignine (ADL) according to Van Soest et al., in 1991. Instead, hemicellulose and cellulose were calculated by the difference between NDF and ADF, and ADF and ADL. Gross energy was evaluated by bomb calorimeter (1261 Model; Parr Instrument, Moline, IL, USA).

Cresol concentration determination in samples was performed following the procedure suggested by Birkett et al., 1995.'

It emerged from the sperimentation that collected samples during 48 hours fermentation shown a significant reduction in cresol production. Just after a 31 hours fermentative process, the presence of pediocin A in the treatment called BAC+ (in which treatment a producer strain was used) led to a cresol, oncogenic substance, production reduction, (-73.30%; P<0.05) compared to treatment with isogenetic mutant (BAC-, (in which treatment it was used a non producer and sensitive strain), while after 48 hours cresol concentration reduction the presence was attested on still more significant values: -87.65%; P, 0.05. The addition, of pediocin A to fermentation liquid at a final concentration of 160 AU/ml, led to a very strong putrescine production during the first 4 hours (+203.40% vs BAC-; P,0.001) after which it was stabilized on these values until the end of the test. Spermidine concentration after addition of pediocin A to fermentation liquid always attested on higher values compared to reference control (BAC-), even if from high values'recorded on 16th hour (37.58 $\mu$mol/L) more reduced values were reached at the end of the test (8.08 $\mu$mol/L).

In the PROSPECT 1, Figure A, B and C it is reported the effect of pediocin A on polyamines and cresol production, when introduced in fermentation liquid at a final concentration of 160 active units (AU)/ml.

Animals and diet

An in vivo test (72 weaned little pigs, seghers breed) monitored the effects of a diet supplemented with the pediocin A producer strain FBB61 Pediococcus pentosaceus (BAC+) in comparison with the BAC- negative control determined from FBB61-2 *Pediococcus pentosaceus* isogenetic strain. Basic diet was constituted as described in table 1 and 2. From the beginning of the experiment, 56 days lasting, animals (females and gelt males) shown an average live weight (LV) of 7 kg and were 21 days aged. The subjects were divided in 3 groups: 1) CTR control receiving a standard diet, 2) BAC+ receiving coltured FBB61 Pediococcus pentosaceus over a M17 medium (OXOID) (feed/colture broth ratio 2:1), 3) BAC- receiving FBB61-2 Pediococcus pentosaceus grown in the same coltures medium and same ratios. From day 0 to day 35 of the test, the animals were fed ad libitum, then rationed at 9% metabolic live weight. On days 0, 14, 35, 56 animals were weighed seperetely and growth performances collected, as well as consumption parameters. Variances study for the performance parameters was developed according to GLM (General Linear Model) using SAS ver 6.12 (SAS Institute). Initial live weight was considered as covariance in order to evaluate live weights and daily increases. Used statistical model was the following:

$$Yij = \mu + \tau i + \beta j \ (xij - x) + \varepsilon ij$$

where: $\mu$ = general average; $\tau$ = animal effect; $\beta$ = correction factor for the co-varied; x = examinations average; $\varepsilon$ = error.

After 35 days test, 6 animals from treatments BAC+ and BAC- were sacrificed for the intestinal morphometric parameters analysis (thickness of small and large intestine's wall, crypts depth, villi thickness, villi length, mucosal tunica, mitotic index, apoptosys index). At the same time, also pancreas was extracted for the same kind of laboratory analysis. The following day, 4 animals of each group were selected in order to test intestinal mucosa functionality, by means of in vivo absorption tests. For this purpose, 2 marker molecules were used: bovine serum albumins (BSA, 500 mg/kg LW) and sodium-fluorescine (Na-f, 9,4 mg/kg LW). Molecules were dissolved in normal saline before being introduced directly in the gastric cavity by catheterization. After 0,5, 1, 2, 4, 8 and 24 hr from administration, 4 ml sampling of blood were taken for following analysis.

From performed experimentation, it emerged that the comparison executed between growth performances of two groups receiving two bacterial strains shown interesting aspects (table 3). Just after 35 days from the beginning of the test, even without significant weight differences, animals receiving FBB61 Pediococcus pentosaceus shown a better use of feeds, as indicated from food'capability ratios (FCR), when compared to their inner controls (BAC-) (1.58 BAC+ vs 1.G8 BAC-, P=0.09). As the food capability did not differ during the first investigation period (0-14 days), the best registered trend after 35 days is to be ascribed to a best FCR during the period from day 14th to 35th: BAC+ -5.6% (P=0.08) vs BAC-.

Histological tests shows deeper crypt in animals belonging to BAC+ group than those of BAC- group, both in the proximal and medium intestinal jejunum, suggesting in these regions a higher cell proliferation rate. Conversely, histological test of medial intestine

revealed a lower mitotic index in animal receiving FBB61 *Pediococcus pentosaceus* (ATCC 43200) compared to animals receiving FBB61-2 Pediococcus pentosaceus. This date explains how the greater depth of crypts is due to a greater secretion activity of cells therein, rather to their greater proliferation. Relative measurements to intestinal villi structures shown (Table 4 and 5): 1) a potential greater length of villi in intestinal proximal and medium jejunum; 2) a significant increase of brush border thickness, constituted of micro-villi at enterocytes luminal apex; 3) a significantly thicker mucous tunica in comparison with BAC-group animals, both on a proximal and medium jejunum level; a lower cell division rate on a medium jejunum level. The latter remark combined to the potential greater thickness of villi may indicate a reduced intestinal cell turnover, which would not affect the mucosa integrity, however, as demonstrated by in vivo permeability tests. Increasing of brush border thickness of enterocytes, with the potential greater thickness of villi preludes an increased absorbing surface. On a caecal level, a treatment with FBB61 Pediococcus pentosaceus led to a reduced thickness of mucous tunica without affecting other structures, as well as to unchanged pancreatic histological parameters.

Table 1: control diet used into in vivo test. Treated group diets, starting from the same base, included an addition in a 1:2 ratio of colture broth containing FBB61 Pediococcus pentosaceus (BAC+) or FBB61-2 Pediococcus pentosaceus (BAC-).

Ingredients (g/kg feed)
Corn 327.2
Soybean flour 210.0
Soybean oil 20.0
Barley 70.0
Flaked barley 250.0
Fish meal 30.0
Powdered serum 50.0
L-lysine 5.0
DL methionine 1.0
Calcium carbonate 3.0
Calcium phosphate 27.0
Sodium chloride 1.8
Vitaminic mineral mix 5.0
SUPPLIES (per kg of feed)
Proteins 179.4
Fats 52.9
Fibre 47.9
Ashes 66.2
Starch 418.5
Digestible energy (MJ) 0.811
Net energy (MJ) 0.612
Supplies of vitaminic mineral mix (per kg of feed)
Vitamin A (UI) 15,000
Vitamin D3 (UI) 1,700
Vitamin E (mg) 35
Fe 200
Cu 170
Zn 200

Table 2. Diet amino acid profile.

|  | Total AA % | Digestible AA % |
|---|---|---|
| Met + Cys | 0.73 | 0.63 |
| Lysine | 1.37 | 1.24 |
| Threonine | 0.71 | 0.60 |
| Tryptophan | 0.23 | 0.19 |

In PROSPECT 2, Table 3 productive performances of animal are reported. Date are presented as $\pm$ mean S.D. (ADG = average daily increase; FCR= food efficiency). In PROSPECT 3, Table 4 morphometric analysis effected over the intestine are reported. Date are presented as $\pm$ mean SEM. * P<0.05, ** P<0.01, *** P<0.0001 when compared to BAC= vs BAC- group animals. MI = mitotic index.

In PROSPECT 4, Table 5 morphometric analysis effected over caecum and pancreas are reported. Date are presented as $\pm$ mean SEM. ** P<0.01, when compared to BAC= vs BAC- group animals. MI = mitotic index.

In PROSPECT 4, Figure D a permeability test is reported. Na-f concentration ($\mu$g/ml) in the sampled animals plasma. Date are presented as $\pm$ mean SD.

PEDIOCIN-CELL STRUCTURES INTERACTIONS

FBB61 Pediococcus pentosaceus lactic bacterium colture, its pediocin A bacteriocin production and its purification were executed as above discussed. Purificated pediocin A was then labelled using an "ECL protein biotynilation" system (Amersham Pharmacia Biotech, UK) following producer's instructions. Labelled protein detection- was performed using Streptavidin horseradish peroxidase conjugated (HRP) for the detection of the Western blot analysis, or by means of Streptavidin-fluorescein isothiocyanate conjugated (FITC) (Sigma Aldrich, USA) for microscopic examinations.

Pediocin A detection over intestinal mucosa

Swines, rats and mice were sacrificed, small and large intestines were collected and immediately washed in cold PBS solution (pH 7.5). A little portion of jejunum in rat and mouse was reversed, so that the mucosa could be perfectly contacted with PBS solution, while from the swine intestine a 0,25 cm$^2$ area was collected. Thus obtained intestine portions were then dipped for 15 min. in a PBS solution containing pediocin A biotynilated (160 AU/ml). Next, in order to remove the protein non-bonded to the mucosa, tissues were washed 2 times in cold PBS. Intestinal portion were then dipped in 2 ml sterile distilled water to remove labelled protein. Tissues were then eliminated. 2 ml solution were then freeze-dried and re-suspended in 40 $\mu$l for following analysis by SDS-PAGE.

SDS-PAGE and Western blot

Obtained samples were examined by SDS-PAGE according to Laemmli (1970) using 3% and 12% acrylamide stacking gel for running gel. After electrophoresis, the proteins isolated from gel were plotted over a nitrocellulose carrier by using a mini Trans-blot (BIO-RAD). Pediocin A detection was performed by using streptavidin-HRP.

Pediocin-mucus interactions

For this test, II type mucines from swine stomach were used, suspended in PBS solution at a concentration of 1 mg/ml. The same quantity (1 mg/ml) was used and incubated for 15 min. at 37°C in a pediocin A-containing solution (160 AU/ml). Samples were then centrifugated at 8000xg for 15 min. and the obtained pellet was washed 3 times with PBS solution. 3 $\mu$l of Streptavidin-FITC were added to the pellet, in the meantime re-suspended in 1 ml of PBS solution. After further 15 min. incubation at room temperature, samples were again centrifugated and pellet washed 3 times with PBS solution. Swine collected tissues followed the same iter: a 0,25 cm$^2$ area was collected and incubated for 15 min. at room temperature in a biotynilated pediocin A-containing buffer at a concentration of 160 AU/ml. The non-bonded protein was washed away through purges with PBS solution. Thus obtained samples were analyzed by using an TMD Nikon inverted microscope provided with Omega XF100 FITC filter set (Optical-USA). The used negative control and represented by the same samples not incubated with pediocin. This is to exclude label interactions with intestinal structures, independently from the presence of pediocin A.

Electron microscopy

FBB61-2 Pediococcus pentosaceus cells (non producer isomutant strain and sensitive to bacteriocin action) contained in 1 ml of fresh colture were collected by centrifugation and re-suspended in 300 $\mu$l of PBS solution containing biotynilated pediocin A at a concentration of 160 AU/ml. After 2 washes with PBS, labelled streptavidin with golden particles (diameter 5 nm) was added. After 10 min. incubation, cells were washed in PBS. Swines, rats and mice were sacrificed, small and large intestines were collected and immediately washed in cold PBS solution (pH 7.5). A little portion of jejunum in rat and mouse was reversed, so that the mucosa could be perfectly contacted with PBS solution, while from swine intestine a 0,25 cm$^2$ area was collected. Intestine portions thus obtained were then dipped for 15 min. in a PBS solution containing biotynilated pediocin A (160 AU/ml). Next, in order to remove the protein non-bonded to the mucosa, tissues were washed 2 times in cold PBS. At this time, bacterial cells and intestinal tissues were prepared for electron microscope analysis (Jeol JEE 1200 EXII, Jeol LTD, Tokyo, Japan, at 80 kV). Tissues were also prepared and analyzed by means of Philips XL 30 ESEM scanning electron microscope.

From the performed experimentation, it has been found that from fluorescence microscope examinations it is evident that pediocin A interacts with mucus. The negative control does not show any fluorescence, which means that streptavidin-FITC does not remain attached to mucus. Pediocin A recovered from mucus has pointed but the maintenance of an excellent antibacterial action, as shown from the presence of inhibition halos in petri dishes previously seeded with FBB61-2 Pediococcus pentosaceus sensitive strain. The mechanism of interaction between pediocin A and mucus is not known yet, but the bacteriocin remains attached also when two mucopolysaccharide structures are completely saturated by lectins. Protein connection detections to intestinal tissues did not display any kind of specific bond with delimitated region by intestine. Conversely, pediocin A is capable to bond to any structures (large or small intestine) with no respect of 3 tested animal species (swine, rat, mouse). An aspecific interaction but in any case effective is further shown between pediocin A and bacterial strain sensitive to its FBB61-2 Pediococcus pentosaceus action. Already after 5 minutes co-incubation, pediocin A molecules are detected close to bacterium cell membrane, both in combinations and single molecules. Electron and scanning microscope examinations shown the presence of pediocin A labelled with streptavidin-gold. More particularly, also in this case pediocin A seems to remain entrapped in the mucus covering gastrointestinal structures, giving protection against GRAM+ pathogens thanks to its preserved antibacterial activity.

Pediocin A has been further characterized as discussed in following tests #1, #2 and #3.

**Test # 1: purification of pediocin A produced by FBB61 P. pentosaceus through tangential flow filtration (TFF).**

In this test a new method of purification of pediocin A was tested by tangential flow filtration technique (TFF). In the usual filtration, a liquid flow to be filtered has a perpendicular direction based on filtering membrane surface, whereas in the TFF the flow runs driven by a pump, in a tangential direction based on filtering membrane. Fluid components which are held an do not pass-through the membrane, as is the case with TFF, do not accumulate over filter surface but they are transported by the flow, preventing in this way the pore obstruction of the membrane.

In the filter modulus a separation between filtrate and retentate occurs; the filtrate is collected, while the retentate is re-introduced in the supply vessel to cyclically proceed the filtration and to increase yields in terms of solute purification and concentration to be recovered.

Colture broth to be subjected to tangential flow filtration (TFF) was obtained by incubating FBB61 P. pentosaceus in a M17 broth + 1% (w/v) glucose at 39°C for. 20 hours at 38°C. At the end of the incubation, the colture broth was centrifugated at 10,000 rpm for 5 minutes, the supernatant collected and filtered (Stericup, Millipore Corporation, Bedford, Massachussets) through Durapore® membranes with 0,45 mm pore diameter. For TFF, a flat filtering modulus was used (Pellicon® XL filter, Millipore Corporation, Bedford, MS, USA), with a modified polyether sulfone Biomax® membrane and NMWL (Nominal Molecular Weight Limit) of 100 kDa (Fig. 15).

TFF filtering modulus was prepared according to producer's instructions. Filtered colture broth was placed in the filtering modulus by means of a peristaltic pump (Minipuls 3, Gilson Italia Srl, Milan, Italy) at a 20 ml/min flow. Filtered colture broth, filtrate and preparation solutions were held in an ice bath throughout the filtration lenght. After reducing the supply solution volume (2,000 ml) at about a quarter of initial volume (530 ml), TFF was interrupted and the two solutions thus obtained, the supply solution, that is retentate (R2), and the filtrate (F2) were frozen at -20°C.

**TEST # 1 RESULTS**

By using a filter with NMWL (Nominal Molecular Weight Limit) of 100 kDa for the tangential flow filtration process, it was believed to recover pediocin A in the filtrate (F), i.e. the crossing filter component. Contrary to all expectations, the inhibiting activity towards the indicator strain (FBB61-2 P. pentosaceus) was found in the retentate (R) containing the substances not able of passing-through the used filtering membrane. By considering that the molecular weight of pediocin A is 80 kDa, probably this result has to be ascribed to the formation of aggregates of pediocin A, given its hydrophobic nature (Piva and Headon, 1994) which is not able to pass through the meshes of the filter used.

The purification of 2,000 ml colture broth with the tangential flow filtration method enables to reach a high efficiency purification (3,55 times the initial colture). The specific activity, hence the ratio between arbitrary units and proteinaceous content (AU/mg) of the sample presents a value of 21.88 AU/mg vs initial 6.16 AU/mg. Further, the total recover of the activity initially present in the filtered supernatant of the existent colture broths inside the initial colture broth (100%) was of particular importance, whereas previous purification methodologies also led to an average diminution of about 20-30% in the first purification steps.

The purification by tangential flow filtration requires considerably lower periods compared to purification with conventional method, by thus facilitating manual procedures and lowering possible error sources and so losses of pediocin A content, and it proves to be a valid purification method in expectation of high quantity production of bacteriocins, particularly of pediocin A.

**Test # 2: determination of pediocin A thermal sensitivity**

Pediocin A was obtained by incubating FBB61 P. pentosaceus in a M17 broth + 1% (w/v) glucose at 39°C for 20 hours at 38°C. At the end of the incubation, the colture broth was centrifugated at 10,000 rpm for 5 minutes, the supernatant collected and filtered (Stericup, Millipore Corporation, Bedford, Massachussets) through Durapore® membranes with 0,45 mm pore diameter. Further, the filtered supernatant was concentrated through dialysis against polyethylene glycol of 20000 Da molecular weight (PEG 20000, Sigma Chemical, St. Louis, MO, USA). For this operation, dialysis tubes with 12000/14000 MW cut-off (Spectra/Pro 4, Spectrum, Houston, Texas, USA) were employed. The process was carried out at a temperature of 4°C for a whole night. To adjust pH values close to neutral, the dialysed thus obtained was subjected to a second dialysis against a PBS solution (137 mM NaCl + 2,7 mM KCl + 4,3 mM $Na_2HPO_4$. $7H_2O$ + 1,4 mM $KH_2PO_4$) for 24 hours at 4°C, and designated D1+PBS.

Thermal sensitivity of pediocin A thus produced and semi-purified was evaluated by exposing aliquots of D1+PBS preparation at increasing temperatures (45°C, 55°C, 65°C, 75°C, 85°C) for increasing time intervals (5 min., 15 min., 30 min., 45 min., 60 min.). Four aliquots of 1 ml pediocin A D1+PBS preparation were distributed in eppendorf tubes and dipped in water for each temperature-time combinations. After exposition to the temperature for the established time intervals, each sample was freezed at - 20°C before evaluating pediocin A activity by wells technique.

**TEST #2 RESULTS**

The performed test allowed to confirm thermal sensitivity of pediocin A, which does not show any inhibition of FBB61-2 P. pentosaceus indicator strain in the activity evaluation by means of wells technique, if it has been previously subjected to a treatment at 65°C. for 45 minutes (Tab. 6). Treatments at 65°C for lower time intervals than 45 minutes and treatments at lower temperatures than 65°C for all considered time intervals did not decrease Pediocin A activity. Higher temperatures treatments than 65°C, for each considered time interval decreased the inhibiting activity of pediocin A below detection limit of the selected technique.

Thanks to its characterization, pediocin A may be used in all those preparations whose production technology involves the attainment of high working temperatures, not higher than 65° for not longer than 30 minutes. Tab. 6. AU/ml detected with wells technique over pediocin A samples exposed to thermal treatment.

|          | 45°C  | 55°C.  | 65°C  | 75°C | 85°C |
|----------|-------|--------|-------|------|------|
| 5 min.   | 1,600 | 1,600  | 1,600 | 0    | 0    |
| 15 min.  | 1,600 | 1,600  | 1,600 | 0    | 0    |
| 30 min.  | 1,600 | 1,600  | 1,600 | 0    | 0    |
| 45 min.  | 1,600 | 1,600  | 0     | 0    | 0    |
| 60 min.  | 1,600 | 1, 600 | 0     | 0    | 0    |

**Test #3: contact tests between pediocin A and Clostridium perfringens**

Pediocin A antimicrobial activity over the C. perfringes growth was evaluated by exposing Clostridium perfringens bacterium to increasing quantities (20, 40, 80, 160, 320 AU/500µl) of bacteriocin itself for a predetermined time interval of 60 minutes. For this test, pediocin A preparation obtained by tangential filtration was used, as in the above discussed "Test #2".

**TEST #3 RESULTS**

It has been detected a significant reduction (P<0.05) of C. perfringens cells number recovered at the end of 60 minutes of contact with the solution containing a greater quantity of pediocin A (320 AU/500 µl) based on cells number recovered after 60 minutes residence time in a normal saline. Treated sample with 320 AU/500µl shows a reduction of colonies-forming units (CFU) recovered at 67% based on a non-treated sample. Such reduction is not present in other examined samples.

**Tab. 7.** Contact test results between Clostridium perfringens and pediocin A. AU = arbitray units of pediocin A existent in 500µl; * = no.=3; to different letters correspond significantly different results (P<0.05).

| AU | UFC/500µL* |
|----|------------|
| 0  | 8.00±2.83a |

(continued)

| AU | UFC/500$\mu$L* |
|-----|-----------------|
| 320 | 2.67$\pm$0.58[b] |
| 160 | 9.50$\pm$0.71[a] |
| 80 | 8.00$\pm$3.46[a] |
| 40 | 10.00$\pm$1.73[a] |
| 20 | 12.00$\pm$2.83[a] |

Further, from the contact tests by wells technique it has been possible to evaluate the Clostridium perfringens bacterium sensitivity against pediocin A antimicrobial action, by comparing it to that of FBB61-2 Pediococcus pentosaceus standard indicator strain. This text allowed to establish that C. perfringens sensitivity is 160-fold higher than FBB61-2 P. pentosaceus.

## Claims

1. A composition comprising. Pediocin A and its bacterial producer strains, wherein the bacterial strain is selected from at least one of:

    *Pediococcus pentosacaus* FBB61, ATCC 43200
    *Pediococcus pentosaceus* FBB63;
    *Pediococcus pentosaceus* L7230, ATCC 43201
    for use as a medicament.

2. The composition according to claim 1, for use as a diet additive.

3. The use of a composition according to claim 1 for the manufacture of a medicament for the prevention and profilaxis of *Clostridia* infection; preferably of *Clostridium perfringens* infections.

4. The use of a composition according to claim 1 for the manufacture of a medicament for the prevention and profilaxis of cresol due skin and epatic tumors.

5. The use of a composition according to claim 1 for the manufacture of a medicament for the treatment of cresol due depression of animal growth; preferably, in swines.

6. The use of a composition according to claim 2, for the manufacture of a diet additive for increasing the production of the energetic sources for gastroenteric structures, said energetic sources being represented by putrescine and spermidine.

7. The use of a composition according to claim 2 for the manufacture of a diet additive for increasing the epithelial surface of intestinal wall deputed to the absorbtion of nutrients.

8. The use of a composition according to claim 2 for the manufacture of a diet additive for increasing the length of villi in intestinal, proximal and medium jejunum.

9. The use of a composition according to claim 2 for the manufacture of a diet additive for increasing the thickness of brush border, constituted of microvilli at enterocytes luminal apex.

10. The use of a composition according to claim 2 for the manufacture of a diet additive for increasing the thickness of mucous tunica, both on a proximal and medium jejunum level.

## Patentansprüche

1. Zusammensetzung, umfassend Pediocin A und seine bakteriellen Herstellungsstämme, wobei der Bakterienstamm aus mindestens einem von

Pediococcus pentasaceus FBB61, ATCC 43200,
Pediococcus pentasaceus FBB63,
Pediococcus pentasaceus L7230, ATCC 43201,
ausgewählt wird, zur Verwendung als Arzneimittel.

**2.** Zusammensetzung gemäß Anspruch 1, zur Verwendung als Ernährungsergänzungsmittel.

**3.** Verwendung einer Zusammensetzung gemäß Anspruch 1 bei der Herstellung eines Arzneimittels für die Prävention und Prophylaxe von *Clostridia*-Infektionen, vorzugsweise von *Clostridium perfringens*-Infektionen.

**4.** Verwendung einer Zusammensetzung gemäß Anspruch 1 für die Herstellung eines Arzneimittels für die Prävention und Prophylaxe von Kresol-verschuldeter Haut und epatischen Tumoren.

**5.** Verwendung einer Zusammensetzung gemäß Anspruch 1 für die Herstellung eines Arzneimittels für die Behandlung von Kresol-verschuldeter Verminderung des Tierwachstums; vorzugsweise in Schweinen.

**6.** Verwendung einer Zusammensetzung gemäß Anspruch 2 für die Herstellung eines Ernährungsergänzungsmittels zum Vergrößern der Herstellung der Energiequellen der Magen- und Darmstrukturen, wobei diese Energiequellen durch Putrescin und Spermidin dargestellt werden.

**7.** Verwendung einer Zusammensetzung gemäß Anspruch 2 für die Herstellung eines Ernährungsergänzungsmittels zum Vergrößern der epithelialen Oberfläche, die der Aufnahme von Nährstoffen gewidmet ist.

**8.** Verwendung einer Zusammensetzung gemäß Anspruch 2 für die Herstellung eines Ernährungsergänzungsmittels zum Vergrößern der Länge der Villi im Darm, proximalem und medialem Jejunum.

**9.** Verwendung einer Zusammensetzung gemäß Anspruch 2 für die Herstellung eines Ernährungsergänzungsmittels zum Vergrößern des Bürstensaums, bestehend aus Microvilli am luminalen Apex der Enterocyten.

**10.** Verwendung einer Zusammensetzung gemäß Anspruch 2 für die Herstellung eines Ernährungsergänzungsmittels zum Vergrößern der Dicke der mukösen Tunika, sowohl auf der Ebene des proximalen als auch des medialen Jujunums.

**Revendications**

**1.** Composition comprenant de la pédiocine A et sa souche productrice bactérienne, où la souche bactérienne est sélectionnée parmi au moins l'une des souches suivantes :

*Pediococcus pentosaceus* FBB61, ATCC 43200,
*Pediococcus pentosaceus* FBB63.
*Pediococcus pentosaceus* L7230, ATCC 43201,
pour une utilisation en tant que médicament.

**2.** Composition selon la revendication 1, pour une utilisation en tant qu'additif alimentaire.

**3.** Utilisation d'une composition selon la revendication 1, pour la fabrication d'un médicament destiné à la prévention et à la prophylaxie des infections par *Clostridia,* de préférence des infections par *Clostridium perfringens.*

**4.** Utilisation d'une composition selon la revendication 1, pour la fabrication d'un médicament destiné à la prévention et à la prophylaxie de tumeurs cutanées et hépatiques dues au crésol.

**5.** Utilisation d'une composition selon la revendication 1, pour la fabrication d'un médicament destiné au traitement de la dépression de la croissance animale due au crésol, de préférence chez des porcs.

**6.** Utilisation d'une composition selon la revendication 2, pour la fabrication d'un additif alimentaire destiné à augmenter la production des sources énergétiques des structures gastroentériques, lesdites sources énergétiques étant représentées par la putrescine et la spermidine.

**7.** Utilisation d'une composition selon la revendication 2, pour la fabrication d'un additif alimentaire destiné à augmenter la surface épithéliale de la paroi intestinale dédiée à l'absorption des nutriments.

**8.** Utilisation d'une composition selon la revendication 2, pour la fabrication d'un additif alimentaire destiné à augmenter la longueur des villosités dans le jéjunum proximal et moyen intestinal.

**9.** Utilisation d'une composition selon la revendication 2, pour la fabrication d'un additif alimentaire destiné à augmenter l'épaisseur de la bordure en brosse, constituée de microvillosités au niveau de l'apex luminal des entérocytes.

**10.** Utilisation d'une composition selon la revendication 2, pour la fabrication d'un additif alimentaire destiné à augmenter l'épaisseur de la tunique muqueuse, à la fois au niveau du jéjunum proximal et moyen.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9729645 A

**Non-patent literature cited in the description**

- **Vervaeke et al.** *methodology,* 1989